# EUROPÄISCHE PATENTSCHRIFT

(11) **EP 0 970 949 B1**
(45) Veröffentlichungstag und Bekanntmachung des Hinweises auf die Patenterteilung: **20.08.2003**
(21) Anmeldenummer: 99111564.3
(22) Anmeldetag: 15.06.1999
(51) Int. Cl.: C07D 219/06, C07D 219/02, A61K 31/435

(54) **Acridinderivate**
Acridine derivatives
Dérivés de l'acridine

(30) Priorität: 06.07.1998 DE 19830105
(43) Veröffentlichungstag der Anmeldung: 12.01.2000
(60) Teilanmeldung: 03013144.5
(73) Patentinhaber: Grünenthal GmbH, 52078 Aachen (DE)
(72) Erfinder: Pütz, Claudia Katharina, Dr., 52349 Düren (DE); Strassburger, Wolfgang Werner Alfred, Prof. Dr., 52146 Würselen (DE); Zimmer, Oswald, Dr., 52146 Würselen (DE); Englberger, Werner Günter, Dr., 52223 Stolberg (DE)

(56) Entgegenhaltungen:
- EP-A- 0 780 369
- EP-A- 0 799 819
- EP-A- 0 864 559
- EP-A- 0 922 703
- US-A- 3 652 589
- K FLICK ET AL: "Untersuchungen zur chemischen Struktur und analgetischen Wirkung von phenylsubstituierten Aminomethylcyclohexanolen" ARZNEILMITTEL. FORSCH, DRUG RES., Bd. 28, Nr. 1a, 1978, Seiten 107-113, XP000608150

## Beschreibung

Die Erfindung betrifft Acridinderivate der allgemeinen Formel I, 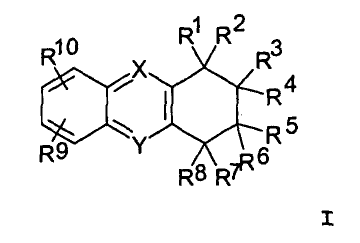 oder pharmazeutisch verwendbare Salze davon, sowie Verfahren zu ihrer Herstellung und deren Verwendung als Arzneimittel.

Klassische Opioide wie Morphin sind bei der Therapie starker bis stärkster Schmerzen gut wirksam. Ihr Einsatz wird jedoch durch die bekannten Nebenwirkungen z. B. Atemdepression, Erbrechen, Sedierung, Obstipation und Toleranzentwicklung limitiert. Außerdem sind sie bei neuropathischen oder inzidentiellen Schmerzen, unter denen insbesondere Tumorpatienten leiden, weniger wirksam.

Opioide entfalten ihre analgetische Wirkung durch Bindung an membranständige Rezeptoren, die zur Familie der sogenannten G-Protein-gekoppelten Rezeptoren gehören. Die biochemische und pharmakologische Charakterisierung von Subtypen dieser Rezeptoren zeigt, daß subtypenspezifische Opioide über ein anderes Wirkungs-/Nebenwirkungsprofil als z. B. Morphin verfügen. Während Morphin selektiv an die sogenannten µ-Rezeptoren bindet, wurden die endogenen Enkephaline als δ-selektive Peptide charakterisiert. Weitere pharmakologische Untersuchungen haben inzwischen die Existenz mehrerer Subtypen dieser Opioidrezeptoren (µ₁, µ₂, κ₁, κ₂, κ₃, δ₁ und δ₂) wahrscheinlich gemacht.

Kenntnisse über die physiologische Bedeutung von δ-rezeptorselektiven Substanzen sind wesentlich durch die Entwicklung des nicht-peptidischen Antagonisten Naltrindol erweitert worden. So steht inzwischen fest, daß δ-Agonisten über ein eigenständiges antinociceptives Potential verfügen. Neben einer Vielzahl von tierexperimentellen Studien gibt es auch eine Untersuchung mit dem peptidischen Agonisten D-Alanin²-D-Leucin⁵-Enkephalin (DADL) an Krebspatienten, bei denen Morphin keine analgetische Wirkung mehr hatte. Bei intrathekaler Gabe zeigte DADL einen lang anhaltenden analgetischen Effekt. Außerdem unterscheiden sich δ- von µ-Agonisten in ihrer Wechselwirkung mit dem "endogenen Opioidantagonisten" Cholecystokinin (CCK).

Die der Erfindung zugrundeliegende Aufgabe bestand darin, analgetisch wirksame Substanzen zu finden, die eine Affinität zu δ-Opiatrezeptoren zeigen.

Mit den erfindungsgemäßen Acridinderivaten der vorliegenden Erfindung sind diese Anforderungen erfüllt worden. Die neuen Verbindungen zeigen eine deutliche δ-Opiatrezeptor-Affinität.

Gegenstand der Erfindung sind Acridinderivate der allgemeinen Formel I, 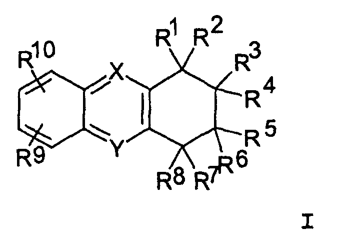 worin
- R¹: A, wenn
- R²: H, OR¹² oder R² und R³ zusammen eine Doppelbindung bilden;
- R³: H oder R³ und R² zusammen eine Doppelbindung bilden;
- R⁴: CH₂NR¹⁴R¹⁵;
- R⁵: H, C₁₋₆-Alkyl;
- R⁶: H, C₁₋₆-Alkyl;
- R⁷: H;
- R⁸: H;
oder
- R³: A, wenn
- R¹: H oder R¹ und R⁴ zusammen eine Doppelbindung bilden;
- R²: H;
- R⁴: H, OR¹² oder R⁴ und R¹ zusammen eine Doppelbindung oder R⁴ und R⁵ zusammen eine Doppelbindung bilden;
- R⁵: H oder R⁵ und R⁴ zusammen eine Doppelbindung bilden;
- R⁶: CH₂NR¹⁴R¹⁵;
- R⁷: H;
- R⁸: H;
oder
- R⁵: A, wenn
- R¹: H;
- R²: H;
- R³: H oder R³ und R⁶ zusammen eine Doppelbindung bilden;
- R⁴: H;
- R⁶: H, OR¹² oder R⁶ und R³ zusammen eine Doppelbindung oder R⁶ und R⁷ zusammen eine Doppelbindung bilden;
- R⁷: H oder R⁷ und R⁶ zusammen eine Doppelbindung bilden;
- R⁸: CH₂NR¹⁴R¹⁵;
und
- A: 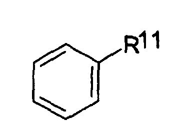
- R⁹, R¹⁰: gleich oder verschieden voneinander H, OH, C₁₋₆-Alkoxy, Cl, F, CN, CF₃, COOH, CONR¹⁷R¹⁸, COOR¹⁶;
- R¹¹: H, OH, C₁₋₆-Alkoxy, O-C₃₋₇-Cycloalkyl; O-Aryl, wobei "Aryl" unsubstituiertes oder ein- oder mehrfach mit OH, F, Cl, CF₃, C₁₋₆-Alkyl, C₁₋₆-Alkoxy, C₃₋₇-Cycloalkyl, C₂₋₆-Alkylen, Heterocyclyl oder Phenyl substituiertes Phenyl oder Naphthyl bedeutet; O-Heterocyclyl, wobei "Heterocyclyl" 5- oder 6-gliedrige gesättigte oder ungesättigte, gegebenenfalls mit einem ankondensierten Arylsystem versehene, heterocyclische Verbindungen bedeutet, die ein oder zwei Heteroatome aus der Gruppe von Stickstoff, Sauerstoff und/oder Schwefel enthalten;
- R¹²: H, C₁₋₆-Alkyl; Aryl, wobei "Aryl" unsubstituiertes oder ein- oder mehrfach mit OH, F, Cl, CF₃, C₁₋₆-Alkyl, C₁₋₆-Alkoxy, C₃₋₇-Cycloalkyl, C₂₋₆-Alkylen, Heterocyclyl oder Phenyl substituiertes Phenyl oder Naphthyl bedeutet; COR¹³;
- R¹³: C₁₋₆-Alkyl; Aryl, wobei "Aryl" unsubstituiertes oder ein- oder mehrfach mit OH, F, Cl, CF₃, C₁₋₆-Alkyl, C₁₋₆-Alkoxy, C₃₋₇-Cycloalkyl, C₂₋₆-Alkylen, Heterocyclyl oder Phenyl substituiertes Phenyl oder Naphthyl bedeutet;
- R¹⁴, R¹⁵: gleich oder verschieden voneinander C₁₋₆-Alkyl; Aryl, wobei "Aryl" unsubstituiertes oder ein-oder mehrfach mit OH, F, Cl, CF₃, C₁₋₆-Alkyl, C₁₋₆-Alkoxy, C₃₋₇-Cycloalkyl, C₂₋₆-Alkylen, Heterocyclyl oder Phenyl substituiertes Phenyl oder Naphthyl bedeutet; C₃₋₇-Cycloalkyl;
- R¹⁶: C₁₋₆-Alkyl; Aryl, wobei "Aryl" unsubstituiertes oder ein- oder mehrfach mit OH, F, Cl, CF₃, C₁₋₆-Alkyl, C₁₋₆-Alkoxy, C₃₋₇-Cycloalkyl, C₂₋₆-Alkylen, Heterocyclyl oder Phenyl substituiertes Phenyl oder Naphthyl bedeutet;
- R¹⁷, R¹⁸: gleich oder verschieden voneinander C₁₋₆-Alkyl; Aryl, wobei "Aryl" unsubstituiertes oder ein-oder mehrfach mit OH, F, Cl, CF₃, C₁₋₆-Alkyl, C₁₋₆-Alkoxy, C₃₋₇-Cycloalkyl, C₂₋₆-Alkylen, Heterocyclyl oder Phenyl substituiertes Phenyl oder Naphthyl bedeutet; und
X gleich N wenn Y gleich C, oder X gleich C wenn Y gleich N
bedeuten, oder pharmazeutisch verwendbare Salze davon.

Bevorzugt sind Verbindungen der allgemeinen Formel I worin R¹⁴, R¹⁵ gleich oder verschieden voneinander C₁₋₆-Alkyl und R¹ bis R¹³, R¹⁶ bis R¹⁸, X und Y die obige Bedeutung oder
R¹¹ OH oder C₁₋₆-Alkoxy und R¹ bis R¹⁰, R¹² bis R¹⁸, X und Y die obige Bedeutung oder
R¹ A, R¹¹ OH oder C₁₋₆-Alkoxy, R¹⁴ und R¹⁵ gleich oder verschieden voneinander C₁₋₆-Alkyl und R² bis R¹⁰, R¹², R¹³, R¹⁶ bis R¹⁸, X und Y die obige Bedeutung oder
R³ A, R¹¹ OH oder C₁₋₆-Alkoxy, R¹⁴ und R¹⁵ gleich oder verschieden voneinander C₁₋₆-Alkyl und R¹, R², R⁴ bis R¹⁰, R¹², R¹³, R¹⁶ bis R¹⁸, X und Y die obige Bedeutung oder
R⁵ A, R¹¹ OH oder C₁₋₆-Alkoxy, R¹⁴ und R¹⁵ gleich oder verschieden voneinander C₁₋₆-Alkyl und R¹ bis R⁴, R⁶ bis R¹⁰, R¹², R¹³, R¹⁶ bis R¹⁸, X und Y die obige Bedeutung besitzen.

Zu weiteren bevorzugten Verbindungen zählen:
*rac*-cis-[3-Dimethylaminomethyl-2-(3-methoxy-phenyl)]-1,2,3,4-tetrahydro-acridin-2-ol, Hydrochlorid,
*rac*-cis-[4-Dimethylaminomethyl-3-(3-methoxy-phenyl)]-1,2,3,4-tetrahydro-acridin-3-ol, Hydrochlorid,
[3-Dimethylaminomethyl-2-(3-hydroxy-phenyl)]-3,4-dihydroacridin-1-en, Hydrochlorid,
*rac*-trans-[3-Dimethylaminomethyl-2-(3-methoxy-phenyl)]-1,2,3,4-tetrahydro-acridin-2-ol, Hydrochlorid,
*rac*-cis-[3-Dimethylaminomethyl-2-(3-hydroxy-phenyl)]-1,2,3,4-tetrahydro-acridin-2-ol, Hydrochlorid,
[1-(3-Methoxy-phenyl)-3,4-dihydro-acridin-2-yl-methyl]-dimethylamin, Hydrochlorid,
[3-(3-Methoxy-phenyl)-1,2-dihydro-acridin-2-yl-methyl]-dimethylamin, Hydrochlorid,
[3-Dimethylaminomethyl-2-(3-methoxy-phenyl)]-3,4-dihydroacridin-1-en, Hydrochlorid,
*rac*-trans-[1-(3-Methoxy-phenyl)-1,2,3,4-tetrahydro-acridin-2-yl-methyl]-dimethylamin, Hydrochlorid,
*rac*-cis-[1-(3-Methoxy-phenyl)-1,2,3,4-tetrahydro-acridin-2-yl-methyl]-dimethylamin, Hydrochlorid,
*rac*-trans-[3-(3-Methoxy-phenyl)-1,2,3,4-tetrahydro-acridin-2-yl-methyl]-dimethylamin, Hydrochlorid,
*rac*-cis-[3-(3-Methoxy-phenyl)-1,2,3,4-tetrahydro-acridin-2-yl-methyl]-dimethylamin, Hydrochlorid,
[3-(2-Dimethylaminomethyl-3,4-dihydro-acridin-1-yl)]-phenol, Hydrochlorid,
[3-(2-Dimethylaminomethyl-1,2-dihydro-acridin-3-yl]-phenol,
*rac*-trans-[3-(2-Dimethylaminomethyl-1,2,3,4-tetrahydroacridin-3-yl)]-phenol,
rac-trans-[3-(2-Dimethylaminomethyl-1,2,3,4-tetrahydroacridin-1-yl)]-phenol, Hydrochlorid,
rac-cis-[2-Dimethylaminomethyl-1-(3-methoxy-phenyl)]-3,3-dimethyl-1,2,3,4-tetrahydro-acridin-1-ol, Hydrochlorid,
3-(2-Dimethylaminomethyl-3,3-dimethyl-3,4-dihydroacri-din-1-yl)-phenol, Hydrochlorid.

Der Ausdruck "C₁₋₆-Alkyl" bedeutet im Rahmen der vorliegenden Erfindung geradkettige oder verzweigte Kohlenwasserstoffe mit 1 bis 6 Kohlenstoffatomen. Beispielhaft seien Methyl, Ethyl, Propyl, Isopropyl, n-Butan, sek-Butyl, tert-Butyl, Neopentyl und n-Hexan genannt.

Der Ausdruck "C₁₋₆-Alkoxy" bedeutet im Rahmen der vorliegenden Erfindung geradkettige oder verzweigte Kohlenwasserstoffe mit 1 bis 6 Kohlenstoffatomen wie oben definiert, die über das Sauerstoffatom gebunden sind.

Der Ausdruck "C₃₋₇-Cycloalkyl" bedeutet im Rahmen der vorliegenden Erfindung gesättigte cyclische Kohlenwasserstoffe mit 3 bis 7 Kohlenstoffatomen. Beispielhaft seien Cyclopropyl, Cyclobutyl, Cyclopentyl, Cyclohexyl oder Cycloheptyl erwähnt.

Der Ausdruck "Aryl" bedeutet im Rahmen der vorliegenden Erfindung unsubstituierte oder ein- oder mehrfach mit OH, F, Cl, CF₃, C₁₋₆-Alkyl, C₁₋₆-Alkoxy, C₃₋₇-Cycloalkyl, C₂₋₆-Alkylen, Heterocyclyl oder Phenyl substituierte Phenyle. Der Ausdruck kann auch Naphthyl bedeuten.

Der Ausdruck "Heterocyclyl" bedeutet im Rahmen der vorliegenden Erfindung 5- oder 6-gliedrige gesättigte oder ungesättigte, gegebenenfalls mit einem ankondensierten Arylsystem versehene, heterocyclische Verbindungen, die ein oder zwei Heteroatome aus der Gruppe von Stickstoff, Sauerstoff und/oder Schwefel enthalten.

Beispielhaft seien für die gesättigten Heterocyclyle Pyrrolidin, Pyran, Thiolan, Piperidin oder Tetrahydroforan aufgeführt.

In der Gruppe der ungesättigten Heterocyclyle seien beispielhaft Furan, Thiophen, Pyrrol, Pyridin, Pyrimidin, Chinolin, Isochinolin, Phthalazin oder Chinazolin aufgeführt.

Diese Verbindungen werden als Analgetika und, ganz allgemein, für alle pathologischen Zustände, die gewöhnlich mit δ-Opiatrezeptoren behandelt werden können, eingesetzt.

Des weiteren sind Verfahren zur Herstellung einer Verbindung der Formel I Erfindungsgegenstand. Zur Herstellung der Verbindungen der Formel I, wobei Derivate ausgeschlossen sind, in denen die Reste R¹ A, R² H, OR¹² oder R² und R³ zusammen eine Doppelbindung bilden, R³ H bzw. R³ und R² zusammen eine Doppelbindung bilden, R⁴ CH₂NR¹⁴R¹⁵, R⁵ und R⁶ C₁₋₆-Alkyl, R⁷ und R⁸ H, die Reste R¹¹, R¹², R¹⁴ und R¹⁵ die gleiche Bedeutung wie oben haben, werden Cyclohexan-Derivate der Formel II, III oder IV, 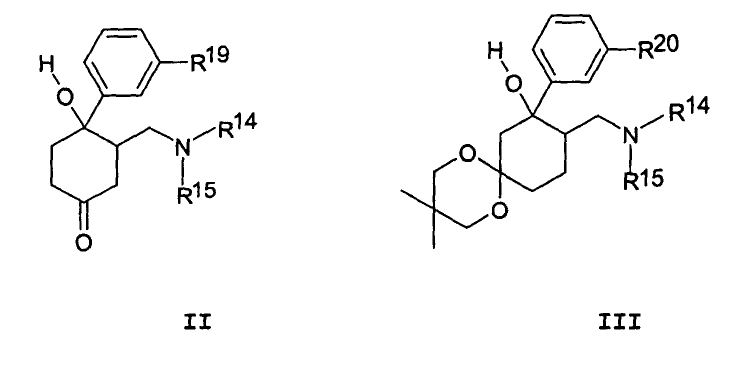 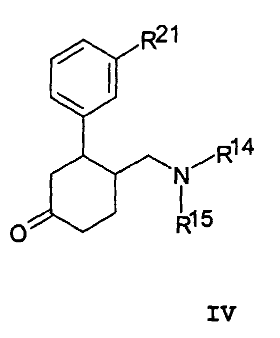 worin R¹⁹, R²⁰ und R²¹ unabhängig voneinander H, C₁₋₆-Alkoxy, O-C₃₋₇-Cycloalkyl; O-Aryl, wobei "Aryl" unsubstituiertes oder ein- oder mehrfach mit OH, F, Cl, CF₃, C₁₋₆-Alkyl, C₁₋₆-Alkoxy, C₃₋₇-Cycloalkyl, C₂₋₆-Alkylen, Heterocyclyl oder Phenyl substituiertes Phenyl oder Naphthyl bedeutet; oder O-Heterocyclyl, wobei "Heterocyclyl" 5- oder 6-gliedrige gesättigte oder ungesättigte, gegebenenfalls mit einem ankondensierten Arylsystem versehene, heterocyclische Verbindungen bedeutet, die ein oder zwei Heteroatome aus der Gruppe von Stickstoff, Sauerstoff und/oder Schwefel enthalten; darstellen, sowie R¹⁴ und R¹⁵ die gleiche Bedeutung wie oben besitzen, mit substituierten 2-Aminobenzaldehyden umgesetzt. Diese Reaktionen werden in einem C₁₋₄-Alkylalkohol in Gegenwart einer Säure aus der Gruppe vorzugsweise von Salzsäure, Phosphorsäure oder Schwefelsäure bei Temperaturen zwischen 20° C und 80° C durchgeführt.

Die Eliminierung der tertiären OH-Gruppe und/oder Spaltung der Methylether-Gruppierung in den erhaltenen Cyclisierungsprodukten wird durch Umsetzung der Produkte mit einer Säure aus der Gruppe vorzugsweise von Ameisensäure, Essigsäure, Bromwasserstoffsäure/Eisessig, Bromwasserstoffsäure oder Methansulfonsäure/Methionin bei Temperaturen zwischen 15° C und 80° C durchgeführt.

Die Einführung des Restes R¹² mit R¹² ungleich Wasserstoff gelingt durch Umsetzung der entsprechenden Cyclisierungsprodukte mit den betreffenden Alkyl- bzw. Arylhalogeniden oder den betreffenden Säurechloriden in Gegenwart einer Base wie beispielsweise Kaliumtertiärbutylat oder Natriumhydrid in einem organischen Lösemittel z. B. Dimethylformamid.

Die Synthese der Cyclohexanone der Formel II mit R¹⁴ und R¹⁵ gleich einer Methylgruppe ist bereits in DE-A 195 47 766 beschrieben.

Cyclohexanone der Formel II mit R¹⁴ und R¹⁵ ungleich einer Methylgruppe und ansonsten der gleichen Bedeutung wie weiter oben beschrieben lassen sich durch Umsetzung eines 1,4-Cyclohexandionmonoethylenketals mit Immoniumsalzen der Formel V, 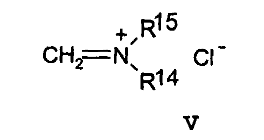 anschließender Reaktion der so erhaltenen Mannich-Basen mit einer metallorganischen Verbindung der Formel VI, 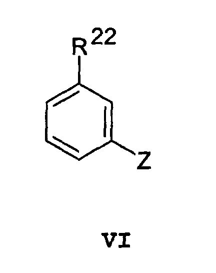 in der Z MgCl, MgBr, MgI oder Lithium bedeutet und R²² die Bedeutung gemäß R¹¹ hat und anschließender Abspaltung der Ketalschutzgruppe mit einer Säure, beispielhaft sei Salzsäure genannt, darstellen.

Die Umsetzung der Mannich-Basen mit einer Grignard-Verbindung der Formel VI in der Z für MgCl, MgBr oder MgI steht, oder mit einer lithiumorganischen Verbindung der Formel VI kann in einem aliphatischen Ether beispielsweise Diethylether und/oder Tetrahydrofuran bei Temperaturen zwischen -70° C und 60° C durchgeführt werden. Die Umsetzung mit einer Grignard-Verbindung der Formel VI kann mit oder ohne Zusatz eines Mitführreagenzes, vorzugsweise 1,2-Dibromethan erfolgen. Lithiumorganische Verbindungen der Formel VI, in der Z Cl, Br oder I bedeutet, lassen sich durch Umsetzung beispielsweise mit n-Butyllithium/Hexan-Lösung durch Halogen-Lithiumaustausch erhalten.

Die Spaltung der Methylether-Gruppierung in den so erhaltenen Cyclohexanderivaten wird durch Umsetzung der Verbindungen mit einer Säure beispielsweise Ameisensäure, Essigsäure, Bromwasserstoffsäure/Eisessig, Bromwasserstoffsäure, Methansulfonsäure/Methionin bei Temperaturen zwischen 15° C und 80° C durchgeführt.

### Cyclohexan-Derivate der Formel III

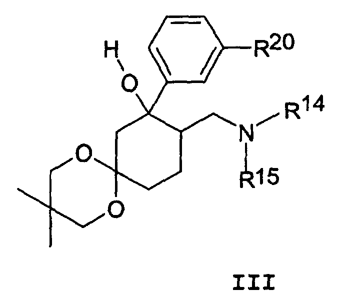

lassen sich durch Reaktion der Mannich-Base der Formel VII, 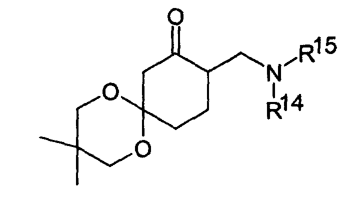 in der R¹⁴ und R¹⁵, die gleiche Bedeutung wie oben haben, mit einer metallorganischen Verbindung der Formel VI, in der Z MgCl, MgBr, MgI oder Lithium bedeuten und R²² die Bedeutung gemäß R¹¹ hat, darstellen.

Die Umsetzung der Mannich-Base der Formel VII mit einer Grignard-Verbindung der Formel VI in der Z MgCl, MgBr oder MgI bedeutet, oder mit einer lithiumorganischen Verbindung der Formel VI kann in einem aliphatischen Ether beispielsweise Diethylether und/oder Tetrahydrofuran bei Temperaturen zwischen -70° C und 60° C durchgeführt werden. Die Umsetzung mit einer Grignard-Verbindung der Formel VI kann mit oder ohne Zusatz eines Mitführreagenzes, vorzugsweise 1,2-Dibromethan erfolgen. Lithiumorganische Verbindungen der VI, in der Z Cl, Br oder I bedeutet, lassen sich durch Umsetzung mit beispielsweise n-Butyllithium/Hexan-Lösung durch Halogen-Lithiumaustausch erhalten.

Die Spaltung der Methylether-Gruppierung in den so erhaltenen Cyclohexanderivaten wird durch Umsetzung der Verbindungen mit einer Säure beispielsweise Ameisensäure, Essigsäure, Bromwasserstoffsäure/Eisessig, Bromwasserstoffsäure, Methansulfonsäure/Methionin bei Temperaturen zwischen 15° C und 80° C durchgeführt. Mannich-Basen der Formel VII mit R¹⁴ und R¹⁵ gleich einer Methylgruppe sind bereits in DE-A 195 25 137 beschrieben. Mannich Basen der Formel VII mit R¹⁴ und R¹⁵ ungleich einer Methylgruppe erhält man durch Umsetzung von 3,3-Dimethyl-1,5-dioxa-spiro[5.5] - undecan-8-on mit Immoniumsalzen der Formel V.

### Cyclohexanone der Formel IV

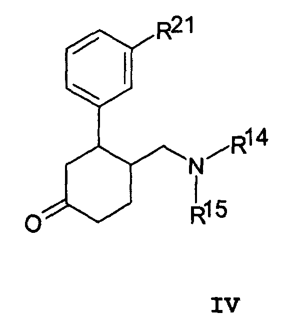

erhält man durch Umsetzung von Cyclohexan-Derivaten der Formel III mit Säuren beispielsweise Salzsäure, Ameisensäure, Essigsäure. Anschließende Hydrierung der so erhaltenen Produkte mit katalytisch aktiviertem Wasserstoff, wobei Platin oder Palladium, absorbiert auf einem Trägermaterial wie Aktivkohle, als Katalysator dienen, führt zu Verbindungen der Formel IV. Die Hydrierung wird in einem Lösemittel wie Essigsäureethylester oder einem C₁₋₄-Alkylalkohol bei Drücken von 0,1 bis 10 bar und Temperaturen von 20° C bis 80° C durchgeführt. Die Spaltung der Methylethergruppe in Verbindungen der Formel IV gelingt durch Umsetzung mit Bromwasserstoffsäure, Bromwasserstoffsäure/Eisessig bei Temperaturen zwischen 20° C und 80° C.

Acridinderivate der allgemeinen Formel I, für die gilt R¹ A, R² H, OR¹² bzw. R² und R³ Teil einer Doppelbindung, R³ H bzw. R³ und R² Teil einer Doppelbindung, R⁴ CH₂NR¹⁴R¹⁵, R⁵ und R⁶ C₁₋₆-Alkyl, R⁷ und R⁸ H, wobei die Reste R¹¹, R¹², R¹⁴ und R¹⁵ die gleiche Bedeutung wie oben besitzen, lassen sich vorzugsweise durch Reaktion von 3,3-Dialkyl-3,4-dihydro-2H-acridin-1-on-Derivaten [vgl. W. Borsche et al., Justus Liebigs Ann. Chem. 550, 160, (1942)] mit Immoniumchloriden der Formel V bei Temperaturen zwischen 20° C und 80° C in einem Lösemittel beispielsweise Acetonitril darstellen. Die daraus resultierenden Mannich-Basen werden mit einer metallorganischen Verbindung der Formel VI in einem aliphatischen Ether z. B. Diethylether und/oder Tetrahydrofuran bei Temperaturen zwischen -70° C und +60° C umgesetzt. Die Eliminierung der tertiären OH-Gruppe und/oder die Spaltung der Methylether-Gruppe in den so erhaltenen Produkten kann mit Säure z. B. Ameisensäure, Essigsäure, Bromwaserstoffsäure/Eisessig, Bromwasserstoffsäure, Methansulfonsäure/Methionin bei Temperaturen zwischen 20° C und 100° C durchgeführt werden. Durch Hydrierung der aliphatischen Doppelbindung in diesen Produkten mit katalytisch aktiviertem Wasserstoff, wobei Platin oder Palladium, absorbiert auf einem Trägermaterial, z. B. Aktivkohle, als Katalysator dienen, erhält man die erfindungsgemäßen Verbindungen der Formel I worin R¹ A, R² H, R³ H, R⁴ (CH₂)N(CH₃)₂, R⁵ CH₃, R⁶ CH₃, R⁷ und R⁸ H bedeuten. Die Reaktion wird in einem Lösemittel wie Essigsäure, Essigsäureethylester oder einem C₁₋₄-Alkylalkohol bei Drücken von 0,1 bis 10 bar und Temperaturen von 20° C bis 80° C durchgeführt.

Die Einführung des Restes R¹² mit R¹² ungleich Wasserstoff gelingt durch Umsetzung der entsprechenden Cyclisierungsprodukte mit den betreffenden Alkyl-, Arylhalogeniden oder den betreffenden Säurechloriden in Gegenwart einer Base wie beispielsweise Kaliumtertiärbutylat, Natriumhydrid in einem organischen Lösemittel vorzugsweise Dimethylformamid.

Die Verbindungen der Formel I lassen sich mit physiologisch verträglichen Säuren wie Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure in an sich bekannter Weise in ihre Salze überführen. Vorzugsweise wird die Salzbildung in einem Lösemittel wie Diisopropylether, Essigsäurealkylester, Aceton und/oder 2-Butanon durchgeführt. Zur Herstellung der Hydrochloride ist Trimethylchlorsilan in wasserhaltiger Lösung besonders geeignet.

### δ-Opiatrezeptor-Bindungsuntersuchungen

Die Untersuchungen zur Bestimmung der Affinität der erfindungsgemäßen Verbindungen der Formel I zum δ-Opiatrezeptor wurde an Hirnmembranhomogenaten (Homogenat von Rattenhirn ohne Cerebellum und Medulla oblongata von männlichen Wistar-Ratten) durchgeführt.

Hierzu wurden jeweils frisch präparierte Rattenhirne unter Eiskühlung in 50 mmol/l Tris-HCl (pH 7,4) homogenisisert und 10 Minuten bei 5.000 g und 4° C zentrifugiert. Nach Dekantieren und Verwerfen des Überstandes, erneutem Aufnehmen und Homogenisieren des Membransedimentes in 50 mmol/l Tris-HCl (pH 7,4) wurde das Homogenat anschließend 20 Minuten bei 20.000 g und 4° C zentrifugiert. Dieser Waschschritt wurde nochmals wiederholt. Danach wurde der Überstand dekantiert und das Membransediment in kaltem 50 mmol/l Tris-HCl, 20 % Glycerin (w/v), 0,01 % Bacitracin (w/v) (pH 7,4) homogenisiert und in Aliquoten bis zur Testung eingefroren. Für die Rezeptorbindungstestung wurden die Aliquote aufgetaut und 1:10 mit dem Bindungstest-Puffer verdünnt. Im Bindungstest wurde als Puffer ein 50 µmol/l Tris-HCl, 5 µmol/l MgCl₂ (pH 7,4), supplementiert mit 0.1 % (w/v) bovinem Serumalbumin, sowie als radioaktiver Ligand 1 nmol [³H]-2-D-Ala-Deltorphin II eingesetzt. Der Anteil an unspezifischer Bindung wurde in Anwesenheit von 10 µmol/l Naloxon bestimmt.

In weiteren Ansätzen wurden die erfindungsgemäßen Verbindungen in Konzentrationsreihen zugegeben und die Verdrängung des radioaktiven Liganden aus seiner spezifischen Bindung ermittelt. Die jeweiligen Dreifachansätze wurden 90 Minuten bei 37° C inkubiert und anschließend zur Bestimmung des an das Membranhomogenat gebundenen radioaktiven Liganden mittels Filtration durch Glasfaserfilter (GF/B) geerntet. Die Glasfaserfilterscheiben wurden getrocknet und deren Radioaktivität nach Zugabe von Szintillator im β-Counter gemessen.

Die Affinität der erfindungsgemäßen Verbindungen zum δ-Opiatrezeptor wurde als IC₅₀ nach dem Massenwirkungsgesetz mittels nichtlinearer Regression berechnet. Aus den IC₅₀-Werten wurden nach der Cheng-Prussoff-Gleichung Kᵢ-Werte berechnet. Die Kᵢ-Werte in Tabelle 1 sind als Mittelwert ±-Standardabweichungen von 3 voneinander unabhängigen Versuchen angegeben.

**Tabelle 1**

| Verbindung | δ-Opiatrezeptorbindung Ki (nM/l) |
|---|---|
| [1-(3-Methoxy-phenyl)-3,4-dihydro-acridin-2-yl-methyl]-dimethylamin, Hydrochlorid | 133 nM ± 15 nM |
| *rac*-trans-[1-(3-Methoxy-phenyl)-1,2,3,4-tetrahydro-acridin-2-yl-methyl]-dimethylamin, Hydrochlorid | 127 nM ± 12 nM |
| [3-(2-Dimethyl-amino-methyl-3,4-dihydro-acridin-1-yl)]-phenol, Hydrochlorid | 3,84 nM ± 1,59 nM |
| *rac*-trans-[3-(2-Dimethylaminomethyl-1,2,3,4-tetrahydro-acridin-1-yl)]-phenol,Hydrochlorid | 4,17 nM ± 0,99 nM |
| rac-cis-[2-Dimethylaminomethyl-1-(3-methoxy-phenyl)]-3,3-dimethyl-1,2,3,4-tetrahydroacridin-1-ol, Hydrochlorid | 60,2 nM ± 14,2 nM |
| 3-(2-Dimethylaminomethyl-3,3-dimethyl-3,4-dihydroacridin-1-yl)-phenol, Hydrochlorid | 29,0 nM ± 3,4 nM |

### Beispiele

Die Ausbeuten der hergestellten Verbindungen sind nicht optimiert.

Alle Temperaturen sind unkorrigiert.

Als stationäre Phase für die Säulenchromatographie wurde Kieselgel 60 (0.040 - 0.063 mm) der Firma E. Merck, Darmstadt, eingesetzt.

Die dünnschichtchromatographischen Untersuchungen wurden mit HPTLC-Fertigplatten, Kieselgel 60 F 254, der Firma E. Merck, Darmstadt, durchgeführt.

Die Mischungsverhältnisse der Laufmittel für alle chromatographischen Untersuchungen sind stets in Volumen/Volumen angegeben.

RT bedeutet Raumtemperatur; Schmp. bedeutet Schmelzpunkt, die Angabe Ether bedeutet Diethylether.

Soweit nicht anders angegeben, wurde Petrolether mit dem Siedebereich von 50°C - 70°C benutzt.

### Beispiel 1

### rac-cis-[3-Dimethylaminomethyl-2-(3-methoxy-phenyl)]-1,2,3,4-tetrahydro-acridin-2-ol, Hydrochlorid und rac-cis-[4-Dimethylaminomethyl-3-(3-methoxy-phenyl)]-1,2,3,4-tetrahydro-acridin-3-ol, Hydrochlorid

4,8 g *rac*-cis-[3-Dimethylaminomethyl-4-hydroxy-4-(3-methoxy-phenyl)]-cyclohexanon und 6,0 g 2-Aminobenzaldehyd als Hydrochlorid wurden bei 20° C unter Stickstoff in 200 ml Methanol gelöst. Anschließend erwärmte man das Reaktionsgemisch auf 80° C und versetzte es bei dieser Temperatur mit 20 ml 1 N Salzsäure. Nach weiteren 48 Stunden kühlte man die Reaktionslösung auf 0° C ab, versetzte mit 200 ml Essigsäureethylester und alkalisierte mit gesättigter Natriumhydroxidlösung. Die wäßrige Phase extrahierte man dreimal mit jeweils 100 ml Essigsäureethylester, trocknete die vereinigten organischen Phasen über Magnesiumsulfat und engte das Gemisch im Vakuum ein. Der Rückstand wurde säulenchromatographisch mit Essigsäureethylester/Methanol im Verhältnis 4/1 als Elutionsmittel gereinigt. Die erste Fraktion enthielt 1,2 g *rac*-cis-[4-Dimethylaminomethyl-3-(3-methoxy-phenyl)]-1,2,3,4-tetrahydro-acridin-3-ol Base als amorphen beigen Feststoff. Zur Darstellung des Hydrochlorids wurde der Feststoff unter Erwärmen in 50 ml Aceton gelöst und mit einer äquimolaren Menge Trimethylchlorsilan und Wasser versetzt. Es wurden 1,0 g *rac*-cis-[4-Dimethylaminomethyl-3-(3-methoxy-phenyl)]-1,2,3,4-tetrahydro-acridin-3-ol, Hydrochlorid (32 % d. Th.) in Form weißer Kristalle erhalten; Schmp.: 175° C bis 180° C.

Die zweite Fraktion lieferte 2,0 g rac-cis-[3-Dimethylaminomethyl-2-(3-methoxy-phenyl)]-1,2,3,4-tetrahydro-acridin-2-ol Base gleichfalls als amorphen, beigen Feststoff. Durch Umsetzung des Feststoffs mit einer äquimolaren Menge Trimethylchlorsilan und Wasser in 200 ml Aceton erhielt man 1,9 g (61,3 % d. Th.) rac-cis-[3-Dimethylaminomethyl-2-(3-methoxy-phenyl)]-1,2,3,4-tetrahydro-acridin-2-ol, Hydrochlorid in Form weißer Kristalle (Schmp.: 181° C bis 183° C).

### Beispiel 2

### rac-trans-[3-Dimethylaminomethyl-2-(3-methoxy-phenyl)]-1,2,3,4-tetrahydro-acridin-2-ol, Hydrochlorid und rac-cis-[3-Dimethylaminomethyl -2-(3-hydroxy-phenyl)]-1,2,3,4-tetrahydro-acridin-2-ol, Hydrochlorid

Unter Einsatz der Cyclohexanone:
*rac*-trans-[3-Dimethylaminomethyl-4-hydroxy-4-(3-methoxyphenyl)]-cyclohexanon und
*rac*-cis-[3-Dimethylaminomethyl-4-hydroxy-4-(3-hydroxyphenyl)]-cyclohexanon
anstelle von *rac*-cis-[3-Dimethylaminomethyl-4-hydroxy-4-(3-methoxy-phenyl)]-cyclohexanon in Beispiel 1 erhielt man unter Verwendung der in Beispiel 1 beschriebenen Verfahrensweise:
*rac* -trans - [3 -Dimethylaminomethyl -2 - (3 -methoxy-phenyl)]-1,2,3,4-tetrahydro-acridin-2-ol, Hydrochlorid
   (Schmp.: 186° C - 190° C).
*rac*-cis-[3-Dimethylaminomethyl-2-(3 -hydroxy-phenyl)]-1,2,3,4-tetrahydro-acridin-2-ol, Hydrochlorid
   (Schmp.: > 250° C)

Eine *rac*-cis-[4-Dimethylaminomethyl-3-(3-methoxy-phenyl)]-1,2,3,4-tetrahydro-acridin-3-ol Hydrochlorid analoge Verbindung wurde in beiden Fällen nicht erhalten.

### Beispiel 3

### [3-Dimethylaminomethyl-2-(3-hydroxy-phenyl)]-3,4-dihydroacridin-1-en, Hydrochlorid und [3-Dimethylaminomethyl-2-(3-methoxy-phenyl)]-3,4-dihydro-acridin-1-en, Hydrochlorid

3,65 g *rac*-cis-[3-Dimethylaminomethyl-2-(3-methoxy-phenyl)]-1,2,3,4-tetrahydro-acridin-2-ol Base wurden bei RT mit 20 ml Methansulfonsäure und 2,2 g Methionin versetzt. Das Reaktionsgemisch rührte drei Tage bei 20° C, die Lösung wurde im Vakuum bis zur Trockne eingeengt; der Feststoff wurde in Wasser gelöst, die Lösung mit Essigsäureethylester überschichtet und mit gesättigter Natriumcarbonatlösung alkalisiert. Die wäßrige Phase wurde dreimal mit je 200 ml Essigsäureethylester extrahiert, die vereinigten organischen Phasen über Magnesiumsulfat getrocknet und im Vakuum vom Lösemittel befreit. Der Rückstand wurde säulenchromatographisch mit Essigsäureethylester/Methanol im Verhältnis 6/1 als Elutionsmittel gereinigt. Die erste Produktfraktion enthielt 0,3 g [3-Dimethylaminomethyl-2-(3-methoxy-phenyl)]-3,4-dihydro-acridin-1-en Base. Zur Darstellung des Hydrochlorids wurde der amorphe Feststoff in 50 ml Aceton gelöst und mit einer äquimolaren Menge Trimethylchlorsilan und Wasser versetzt. Man erhielt 0,3 g (7,9% d. Th.) [3-Dimethylaminomethyl-2-(3-methoxy-phenyl)]-3,4-dihydro-acridin-1-en, Hydrochlorid in Form eines beigen, kristallinen Feststoffes (Schmp.: 195°C - 197°C). Die zweite Produktfraktion lieferte 2,2 g 3-Dimethylaminomethyl-2-(3-hydroxy-phenyl)]-3,4-dihydroacridin-1-en Base, die durch Umsetzung mit einer äquimolaren Menge Trimethylchlorsilan/Wasser in 2,1 g (57,5 % d. Th.) der Titelverbindung [3-Dimethylaminomethyl-2-(3-hydroxyphenyl)]-3,4-dihydroacridin-1-en, Hydrochlorid (Schmp.: 200° C bis 204° C) überführt wurde.

### Beispiel 4

### [1-(3-Methoxy-phenyl)-3,4-dihydro-acridin-2-yl-methyl]-dimethylamin, Hydrochlorid und [3-(3-Methoxy-phenyl)-1,2-dihydro-acridin-2-yl-methyl]-dimethylamin, Hydrochlorid

### Stufe 1:

### rac-cis-[9-Dimethylaminomethyl-8-(3-methoxy-phenyl)]-3,3-dimethyl-1,5-dioxa-spiro[5.5)undecan-8-ol

Es wurden 36 g Magnesiumspäne unter Rühren und Überleiten von trockenem Stickstoff in 100 ml absolutem Tetrahydrofuran suspendiert. Anschließend wurden bei 60° C 280 g m-Bromanisol, gelöst in 200 ml absolutem Tetrahydrofuran, hinzugetropft. Nach Beendigung der Bromanisol-Zugabe rührte man die Reaktionsmischung eine weitere Stunde auf 60° C. Anschließend wurden bei 15° C bis 20° C 244 g 9-Dimethylaminomethyl-3,3-dimethyl-1,5-dioxa-spiro[5.5]undecan-8-on in 1000 ml absolutem Tetrahydrofuran gelöst und zugetropft. Das Reaktionsgemisch wurde über Nacht unter Eiskühlung gerührt und unter Eiskühlung mit 1000 ml gesättigter Ammoniumchloridlösung zersetzt. Die wäßrige Phase wurde zweimal mit je 250 ml Ether extrahiert. Die vereinigten organischen Phasen wurden mit gesättigter Natriumchloridlösung gewaschen und über Magnesiumsulfat getrocknet. Nach Abdampfen des Lösemittels im Vakuum versetzte man den Rückstand solange mit Petrolether, bis die Titelverbindung auskristallisierte. Man erhielt 150 g (41 % d. Th.) *rac*-cis-[9-Dimethylaminomethyl-8-(3-methoxyphenyl)]-3,3-dimethyl-1,5-dioxa-spiro[5.5)undecan-8-ol, in Form weißer Kristalle; Schmp.: 91° C bis 93° C.

### Stufe 2:

### [1-(3-Methoxy-phenyl)-3,4-dihydro-acridin-2-yl-methyl]-dimethylamin, Hydrochlorid und [3-(3-Methoxy-phenyl)-1,2-dihydro-acridin-2-yl-methyl]-dimethylamin, Hydrochlorid

18 g des Produktes aus Stufe 1 wurden unter trockenem Stickstoff in 200 ml Methanol gelöst. Das Reaktionsgemisch versetzte man mit 7,7 g 2-Aminobenzaldehyd als Hydrochlorid und erhitzte anschließend auf 80° C. Nach Zugabe von 100 ml 1N Salzsäure rührte die Reaktionslösung acht Tage bei 80° C. Nach Abkühlung auf Raumtemperatur verdünnte man das Reaktionsgemisch mit 200 ml Essigsäureethylester und alkalisiert unter Eiskühlung mit konzentrierter Natriumhydroxidlösung. Die wäßrige Phase extrahierte man dreimal mit je 100 ml Essigsäureethylester, trocknete die vereinigten organischen Phasen über Magnesiumsulfat und engte im Vakuum bis zur Trockne ein. Den Rückstand eluierte und reinigte man säulenchromatographisch an Kieselgel mit Essigsäureethylester/Methanol im Verhältnis 4/1. Die erste Produktfraktion enthielt 5,4 g [3-(3-Methoxy-phenyl)-1,2-dihydro-acridin-2-yl-methyl]-dimethylamin Base in Form beiger Kristalle. Zur Darstellung des Hydrochlorids wurde der Feststoff unter Erwärmen in 200 ml Aceton gelöst und versetzte mit einer äquimolaren Menge Trimethylchlorsilan und Wasser. Man erhielt 5,2 g (28,3 % d. Th.) [3-(3-Methoxy-phenyl)-1,2-dihydro-acridin-2-yl-methyl]-dimethylamin, Hydrochlorid (hellgelbe Kristalle, Schmp.: 201° C bis 204° C). Als zweite Fraktion erhielt man 4,4 g [1-(3-Methoxy-phenyl)-3,4-dihydro-acridin-2-yl-methyl]-dimethylamin Base in Form eines beigen, amorphen Feststoffs. Zur Freisetzung des Hydrochlorids löste man den Feststoff unter Erwärmen in 200 ml Aceton und versetzte mit einer äquimolaren Menge Trimethylchlorsilan und Wasser. Man erhielt 4,2 g (22,90 % d. Th.) [1-(3-Methoxy-phenyl)-3,4-dihydro-acridin-2-yl-methyl]-dimethylamin, Hydrochlorid in Form hellgelber Kristalle, Schmp.: 195° C bis 198° C.

### Beispiel 5

### [3-(2-Dimethylaminomethyl-3,4-dihydro-1-yl)]-phenol, Hydrochlorid

5,4 g [1-(3-Methoxy-phenyl)-3,4-dihydro-acridin-2-yl-methyl]-dimethylamin Base versetzte man bei RT mit 40 ml Methansulfonsäure und 5,4 g Methionin. Das Reaktionsgemisch rührte zehn Tage bei 20° C und wurde im Vakuum bis zur Trockne eingeengt. Der Feststoff wurde in Wasser gelöst, die Lösung mit Essigsäureethylester überschichtet und mit gesättigter Natriumcarbonatlösung alkalisiert. Die wäßrige Phase extrahierte man dreimal mit je 200 ml Essigsäureethylester, trocknete die vereinigten organischen Phasen über Magnesiumsulfat und engte im Vakuum bis zur Trockne ein. Man erhielt 2,4 g [3-(2-Dimethylaminomethyl-3,4-dihydro-acridin-1-yl)]-phenol Base. Das Lösen des gelben, amorphen Feststoffs unter Erwärmen in Aceton und Versetzen mit äquimolarer Menge Trimethylchlorsilan und Wasser lieferte 2,3 g [3-(2-Dimethylaminomethyl-3,4-dihydroacridin-1-yl)]-phenol, Hydrochlorid (48 % d. Th.) in Form hellgelber Kristalle. (Schmp.: > 250° C).

### Beispiel 6

### 3-(2-Dimethylaminomethyl-1,2-dihydro-acridin-3-yl]-phenol

Unter Einsatz von:
[3-(3-Methoxy-phenyl)-1,2-dihydro-acridin-2-yl-methyl]-dimethylamin anstelle von [1-(3-Methoxy-phenyl)-3,4-dihydroacridin-2-yl-methyl]-dimethylamin, erhielt man entsprechend Beispiel 5 unter Verwendung der dort beschriebenen Verfahrensweise:
[3-(2-Dimethylaminomethyl-1,2-dihydro-acridin-3-yl]-phenol (Schmp.: 202° C - 206° C)

### Beispiel 7

### rac-trans-[1-(3-Methoxy-phenyl)-1,2,3,4-tetrahydro-acridin-2-yl-methyl]-dimethylamin, Hydrochlorid und rac-trans-[3-(3-Methoxy-phenyl)-1,2,3,4-tetrahydro-acridin-2-yl-methyl]-dimethylamin, Hydrochlorid

### Stufe 1:

### [4-Dimethylaminomethyl-3-(3-methoxy-phenyl)]-cyclohex-2-en-on

56 g des Produktes aus Stufe 1 gemäß Beispiel 4 wurde unter Rühren in einer Stickstoffatmosphäre in 370 ml Tetrahydrofuran gelöst. Ein Gemisch aus 150 ml konzentrierter Salzsäure und 150 ml Wasser wurde unter Eiskühlung hinzugetropft. Das Reaktionsgemisch rührte zwei Tage bei Raumtemperatur, wurde mit 200 ml Essigsäureethylester verdünnt und mit gesättigter Natriumhydroxidlösung alkalisiert. Die wäßrige Phase extrahierte man dreimal mit je 100 ml Essigsäureethylester, wusch die vereinigten organischen Phasen mit gesättigter Natriumchloridlösung und trocknete über Magnesiumsulfat. Das Lösemittel wurde im Vakuum evaporiert. Das zurückgebliebene Öl wurde in 200 ml Aceton gelöst und mit einer äquimolaren Menge Trimethylchlorsilan und Wasser versetzt. Man erhielt 38,3 g der [4-Dimethylaminomethyl-3-(3-methoxy-phenyl)]-cyclohex-2-en-on als Hydrochlorid in Form hellgelber Kristalle. Zur Freisetzung der Base wurde der Feststoff unter Eiskühlung in Wasser gelöst, mit Essigsäureethylester überschichtet und mit gesättigter Natriumcarbonatlösung alkalisiert. Die wäßrige Phase wurde dreimal mit je 100 ml Essigsäureethylester extrahiert und über Magnesiumsulfat getrocknet. Nach Abdampfen des Lösemittels im Vakuum erhielt man 36 g (88,7 % d. Th.) der Titelverbindung als gelbes Öl.

### Stufe 2:

### rac-[4-Dimethylaminomethyl-3-(3-methoxy-phenyl)]-cyclohexanon

28,5 g des Produktes aus Stufe 1 wurden in 250 ml absolutem Methanol gelöst. Unter Rühren und Überleiten von trokkenem Stickstoff wurden 2,8 g Palladium-Kohle (10 %ig) als Katalysator zugesetzt. Anschließend wurde fünf Stunden bei einem Druck von 0,2 bar und einer Temperatur von 20° C hydriert. Nach Filtration wurde das Lösemittel im Vakuum abgedampft und der Rückstand säulenchromatographisch an Kieselgel mit Essigsäureethylester/Methanol/Diisopropylether im Verhältnis 4/1/5 als Elutionsmittel gereinigt. Als erste Produktfraktion erhielt man 7,2 g (25,4 % d. Th.) *rac*cis-[4-Dimethylaminomethyl-3-(3-methoxy-phenyl)]-cyclohexanon in Form eines Öls. Die zweite Produktfraktion lieferte 7,4 g (26,1 % d. Th.) rac-trans-[4-Dimethylaminomethyl-3-(3-methoxy-phenyl)]-cyclohexanon gleichfalls als Öl.

### Stufe 3:

### rac-trans-[1-(3-Methoxy-phenyl)-1,2,3,4-tetrahydro-acridin-2-yl-methyl]-dimethylamin, Hydrochlorid und rac-trans-[3-(3-Methoxy-phenyl)-1,2,3,4-tetrahydro-acridin-2-yl-methyl]-dimethylamin, Hydrochlorid

2,6 g *rac*-trans-[4-Dimethylaminomethyl-3-(3-methoxy-phenyl)]-cyclohexanon wurden unter Rühren und Überleiten von trockenem Stickstoff in 100 ml Ethanol gelöst. Das Reaktionsgemisch wurde mit 3,2 g 2-Aminobenzaldehyd als Hydrochlorid versetzt und auf 80° C erhitzt. Bei dieser Temperatur wurden 11 ml 1N Salzsäure zugegeben und die Reaktionslösung zwei Tage bei 80° C gerührt. Nach Abkühlung auf Raumtemperatur verdünnte man das Reaktionsgemisch mit 100 ml Essigsäureethylester und alkalisierte unter Eiskühlung mit konzentrierter Natriumhydroxidlösung. Die wäßrige Phase extrahierte man dreimal mit je 100 ml Essigsäureethylester und trocknete die vereinigten organischen Phasen über Magnesiumsulfat. Nach Entfernen des Lösemittels im Vakuum wurde der Rückstand säulenchromatographisch an Kieselgel mit Essigsäureethylester/Methanol im Verhältnis 4/1 gereinigt. Die erste Produktfraktion enthielt 2,1 g *rac*-trans-[3-(3-Methoxy-phenyl)-1,2,3,4-tetrahydro-acridin-2-yl-methyl]-dimethylamin Base in Form beiger Kristalle, die mit einer äquimolaren Menge Trimethylchlorsilan/Wasser in 2 g (52,6 % d. Th.) der Titelverbindung ractrans-[3-(3-Methoxy-phenyl)-1,2,3,4-tetrahydro-acridin-2-yl-methyl]-dimethylamin, Hydrochlorid (hellgelbe Kristalle, Schmp.: 184° C bis 187° C) überführt wurden. Die zweite Fraktion enthielt 0,4 g rac-trans-[1-(3-Methoxyphenyl)-1,2,3,4-tetrahydro-acridin-2-yl-methyl]-dimethylamin Base. Zur Freisetzung des Hydrochlorids wurde der Feststoff unter Erwärmen in 50 ml Aceton gelöst, mit einer äquimolaren Menge Trimethylchlorsilan und Wasser versetzt. Man erhielt 0,4 g (10,5 % d. Th.) des Hydrochlorids *rac*trans-[1-(3-Methoxy-phenyl)-1,2,3,4-tetrahydro-acridin-2-yl-methyl]-dimethylamin, Hydrochlorid, in Form hellgelber Kristalle, Schmp.: 167° C bis 170° C.

### Beispiel 8

### rac -cis-[1-(3 -Methoxy -phenyl)-1,2,3,4-tetrahydro -acridin-2 -yl-methyl]-dimethylamin, Hydrochlorid und rac-cis-[3-(3-Methoxy-phenyl)-1,2,3,4-tetrahydro-acridin-2-yl-methyl]-dimethylamin, Hydrochlorid

Unter Einsatz von:
*rac*-cis-[1-(3-Methoxy-phenyl)-1,2,3,4-tetrahydro-acridin-2-yl-methyl]-dimethylamin
anstelle von
*rac*-trans-[3-(3-Methoxy-phenyl)-1,2,3,4-tetrahydro-acridin-2-yl-methyl]-dimethylamin in Beispiel 7 wurde unter Anwendung der in Beispiel 7 beschriebenen Verfahrensweise erhalten:
*rac*-cis-[1-(3-Methoxy-phenyl)-1,2,3,4-tetrahydro-acridin-2-yl-methyl]-dimethylamin, Hydrochlorid
   (Schmp.: 118° C - 120° C)
*rac*-cis-[3-(3-Methoxy-phenyl)-1,2,3,4-tetrahydro-acridin-2-yl-methyl]-dimethylamin, Hydrochlorid
   (Schmp.: 210°C 213°C)

### Beispiel 9

### rac -trans - [3-(2-Dimethylaminomethyl-1,2,3,4-tetrahydroacridin-1-yl)]-phenol, Hydrochlorid

1 g *rac*-trans-[1-(3-Methoxy-phenyl)-1,2,3,4-tetrahydroacridin-2-yl-methyl]-dimethylamin Base versetzte man bei Raumtemperatur mit 8 ml Methansulfonsäure und 1 g Methionin. Das Reaktionsgemisch rührte zehn Tage bei 20° C, und wurde hiernach im Vakuum bis zur Trockne eingeengt. Den Feststoff löste man in Wasser, überschichtete die Lösung mit Essigsäureethylester und alkalisierte mit einer gesättigten Natriumcarbonatlösung. Die wäßrige Phase extrahierte man dreimal mit je 200 ml Essigsäureethylester, trokknete die vereinigten organischen Phasen über Magnesiumsulfat und engte im Vakuum bis zur Trockne ein. Man erhielt 0,5 g *rac*-trans-[3-(2-Dimethylaminomethyl-1,2,3,4-tetrahydro-acridin-1-yl)]phenol Base. Lösen des gelben, amorphen Feststoffs unter Erwärmen in Aceton und Versetzen mit äquimolarer Menge Trimethylchlorsilan und Wasser, lieferte 0,5 g (52 % d. Th.) *rac*-trans-[3-(2-Dimethyl-aminomethyl-1,2,3,4-tetrahydro-acridin-1-yl)]-phenol Hydrochlorid (hellgelbe Kristalle, Schmp.: 240° C).

### Beispiel 10

### rac-trans-[3-(2-Dimethylaminomethyl-1,2,3,4-tetrahydroacridin-3-yl)]-phenol

Unter Einsatz von:
*rac*-trans-[3-(3-Methoxy-phenyl)-1,2,3,4-tetrahydro-acridin-2-yl-methyl]-dimethylamin
anstelle von
*rac*-trans-[1-(3-Methoxy-phenyl)-1,2,3,4-tetrahydro-acridin-2-yl-methyl]-dimethylamin in Beispiel 9 erhielt man unter Verwendung der in Beispiel 9 beschriebenen Verfahrensweise:
*rac* -trans-[3-(2-Dimethylaminomethyl-1,2,3,4-tetrahydroacridin-3-yl)]-phenol

### Beispiel 11

### 3-(2-Dimethylaminomethyl-3,3-dimethyl-3,4-dihydro-acridin-1-yl)-phenol, Hydrochlorid

### Stufe 1:

### 2-Dimethylaminomethyl-3,3-dimethyl-3,4-dihydro-2H-acridin-1-on

Eine Lösung von 2,25 g 3,3-Dimethyl-3,4-dihydro-2H-acridin-1-on [W. Borsche et al., Justus Liebigs Ann. Chem. 550, 160 (1942)] in 12 ml trockenem Acetonitril versetzte man mit 0,95 g N,N-Dimethylmethylenimmoniumchlorid sowie einem Tropfen Acetylchlorid und rührte das Gemisch drei Tage bei 20° C. Es wurde mit 30 ml dest. Wasser versetzt und zweimal mit Essigsäureethylester extrahiert. Die wäßrige Phase wurde durch Zugabe von festem Kaliumcarbonat (pH∼9) alkalisiert und dreimal mit Dichlormethan extrahiert. Die vereinigten Extrakte wusch man mit gesättigter Natriumchloridlösung, trocknete über Natriumsulfat und engte im Vakuum bis zur Trockne ein. 0,92 g (32,5 % d. Th.) der Titelverbindung in Form eines leicht gelben Öls blieben zurück.

### Stufe 2:

### rac-cis-[2-Dimethylaminomethyl-1-(3 -methoxy-phenyl)]-3,3-dimethyl-1,2,3,4-tetrahydro-acridin-1-ol

Eine Lösung von 0,75 g 3-Bromanisol in 12 ml trockenem Tetrahydrofuran wurde bei -50° C unter Rühren und Überleiten von trockenem Stickstoff tropfenweise mit 2,5 ml einer 1,6 M Lösung von n-Butyllithium in n-Hexan versetzt. Nach beendeter Zugabe wurde noch 30 Minuten weitergerührt und tropfenweise eine Lösung von 0.85 g des Produktes aus Stufe 1 in 2 ml trockenem Tetrahydrofuran hinzugetopft. Nach einer Reaktionszeit von zwei Stunden bei -50° C wurde mit 10 %-iger Salzsäure zersetzt und zweimal mit Essigsäureethylester extrahiert. Die salzsaure Phase alkalisierte man mit Kaliumcarbonat und extrahierte zweimal mit Dichlormethan. Die vereinigten organischen Phasen wurden über Natriumsulfat getrocknet und im Vakuum eingedampft. Der Rückstand wurde säulenchromatographisch an Kieselgel mit Essigsäureethylester/Methanol im Verhältnis 9/1 als Elutionsmittel gereinigt. Man erhielt 0,47 g (40 % d. Th.) der Titelverbindung als viskose Masse.

### Stufe 3

### 3-(2-Dimethylaminomethyl-3,3-dimethyl-3,4-dihydro-acridin-1-yl)-phenol, Hydrochlorid

Ein Gemisch aus 0,39 g des Produkts aus Stufe 2, 2 ml Methansulfonsäure und 0,227 g Methionin rührte neun Tage bei 20° C, dann weitere zehn Tage bei 40° C. Anschließend zersetzte man mit Eis, alkalisierte mit einer gesättigten Natriumhydrogencarbonatlösung und extrahierte dreimal mit Essigsäureethylester. Die Extrakte wurden mit einer gesättigten Natriumchloridlösung gewaschen, über Natriumsulfat getrocknet und im Vakuum weitgehend eingedampft.

Der beim Versetzen des Rückstands mit n-Hexan ausgefallene Feststoff wurde abgetrennt und im Vakuum getrocknet. (Ausbeute: 0,26 g). Den Feststoff löste man unter Erwärmen in einem Gemisch aus 12 ml Aceton, 3,5 ml Tetrahydrofuran und überführte mit einer äquimolaren Menge Trimethylchlorsilan und Wasser in das Hydrochlorid. Man erhielt 0,175 g (44,3 % d. Th.) der Titelverbindung in Form von Kristallen, die bei 240° C unter Zersetzung schmolzen.

## Patentansprüche

1. Acridinderivate der allgemeinen Formel I, 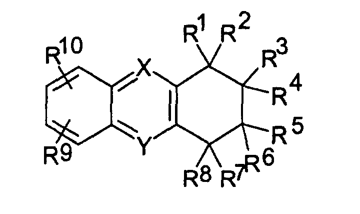 worin
R¹ A, wenn
R² H, OR¹² oder R² und R³ zusammen eine Doppelbindung bilden,
R³ H oder R³ und R² zusammen eine Doppelbindung bilden,
R4 CH₂NR¹⁴R¹⁵;
R⁵ H, C₁₋₆-Alkyl;
R⁶ H, C₁₋₆-Alkyl;
R⁷ H;
R⁸ H;
oder
R³ A, wenn
R¹ H oder R¹ und R⁴ zusammen eine Doppelbindung bilden;
R² H;
R⁴ H, OR¹² oder R⁴ und R¹ zusammen eine Doppelbindung oder R⁴ und R⁵ zusammen eine Doppelbindung bilden;
R⁵ H oder R⁵ und R⁴ zusammen eine Doppelbindung bilden;
R⁶ CH₂NR¹⁴R¹⁵;
R⁷ H;
R⁸ H;
oder
R⁵ A, wenn
R¹ H;
R² H;
R³ H oder R³ und R⁶ zusammen eine Doppelbindung bilden;
R⁴ H;
R⁶ H, OR¹² oder R⁶ und R³ zusammen eine Doppelbindung, R⁶ und R⁷ zusammen eine Doppelbindung bilden;
R⁷ H oder R⁷ und R⁶ zusammen eine Doppelbindung bilden;
R⁸ CH₂NR¹⁴R¹⁵;
und
A 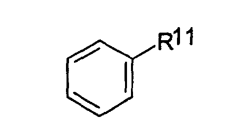
R⁹, R¹⁰ gleich oder verschieden voneinander H, OH, C₁₋₆-Alkoxy, Cl, F, CF₃, CN, COOH, CONR¹⁷R¹⁸, COOR¹⁶;
R¹¹ H, OH, C₁₋₆-Alkoxy, O-C₃₋₇-Cycloalkyl, O-Aryl, wobei "Aryl" unsubstituiertes oder ein- oder mehrfach mit OH, F, Cl, CF₃, C₁₋₆₋ Alkyl, C₁₋₆-Alkoxy, C₃₋₇-Cycloalkyl, C₂₋₆-Alkylen, Heterocyclyl oder Phenyl substituiertes Phenyl oder Naphthyl bedeutet; O-Heterocyclyl, wobei "Heterocyclyl" 5- oder 6-gliedrige gesättigte oder ungesättigte, gegebenenfalls mit einem ankondensierten Arylsystem versehene, heterocyclische Verbindungen bedeutet, die ein oder zwei Heteroatome aus der Gruppe von Stickstoff, Sauerstoff und/oder Schwefel enthalten;
R¹² H, C₁₋₆-Alkyl; Aryl, wobei "Aryl" unsubstituiertes oder ein- oder mehrfach mit OH, F, Cl, CF₃, C₁₋₆-Alkyl, C₁₋₆-Alkoxy, C₃₋₇-Cycloalkyl, C₂₋₆-Alkylen, Heterocyclyl oder Phenyl substituiertes Phenyl oder Naphthyl bedeutet; COR¹³
R¹³ C₁₋₆-Alkyl; Aryl, wobei "Aryl" unsubstituiertes oder ein- oder mehrfach mit OH, F, Cl, CF₃, C₁₋₆-Alkyl, C₁₋₆-Alkoxy, C₃₋₇-Cycloalkyl, C₂₋₆-Alkylen, Heterocyclyl oder Phenyl substituiertes Phenyl oder Naphthyl bedeutet;
R¹⁴, R¹⁵ gleich oder verschieden voneinander C₁₋₆₋Alkyl; Aryl, wobei "Aryl" unsubstituiertes oder ein- oder mehrfach mit OH, F, Cl, CF₃, C₁₋₆-Alkyl, C₁₋₆-Alkoxy, C₃₋₇-Cycloalkyl, C₂₋₆-Alkylen, Heterocyclyl oder Phenyl substituiertes Phenyl oder Naphthyl bedeutet; C₃₋₇-Cycloalkyl;
R¹⁶ C₁₋₆-Alkyl; Aryl, wobei "Aryl" unsubstituiertes oder ein- oder mehrfach mit OH, F, Cl, CF₃, C₁₋₆-Alkyl, C₁₋₆-Alkoxy, C₃₋₇-Cycloalkyl, C₂₋₆-Alkylen, Heterocyclyl oder Phenyl substituiertes Phenyl oder Naphthyl bedeutet;
R¹⁷, R¹⁸ gleich oder verschieden voneinander C₁₋₆-Alkyl; Aryl, wobei "Aryl" unsubstituiertes oder ein- oder mehrfach mit OH, F, Cl, CF₃, C₁₋₆-Alkyl, C₁₋₆-Alkoxy, C₃₋₇-Cycloalkyl, C₂₋₆-Alkylen, Heterocyclyl oder Phenyl substituiertes Phenyl oder Naphthyl bedeutet; und
X gleich N wenn Y gleich C, oder X gleich C wenn Y gleich N
bedeuten, oder pharmazeutisch verwendbare Salze davon.

2. Verbindungen gemäß Anspruch 1, **dadurch gekennzeichnet, daß** R¹⁴ und R¹⁵ gleich oder verschieden voneinander C₁₋₆-Alkyl bedeuten.

3. Verbindungen gemäß Anspruch 1, **dadurch gekennzeichnet, daß** R¹¹ OH oder C₁₋₆-Alkoxy bedeutet.

4. Verbindungen gemäß Anspruch 1, **dadurch gekennzeichnet, daß** R¹ A, R¹¹ OH oder C₁₋₆-Alkoxy, R¹⁴ und R¹⁵ voneinander unabhängig C₁₋₆-Alkyl bedeuten.

5. Verbindungen gemäß Anspruch 1, **dadurch gekennzeichnet, daß** R³ A, R¹¹ OH oder C₁₋₆-Alkoxy, R¹⁴ und R¹⁵ voneinander unabhängig C₁₋₆-Alkyl bedeuten.

6. Verbindungen gemäß Anspruch 1, **dadurch gekennzeichnet, daß** R⁵ A, R¹¹ OH oder C₁₋₆-Alkoxy, R¹⁴ und R¹⁵ voneinander unabhängig C₁₋₆-Alkyl bedeuten.

7. Verbindungen gemäß Anspruch 1 ausgewählt aus der Gruppe:
*rac*-cis-[3-Dimethylaminomethyl-2-(3-methoxy-phenyl)]-1,2,3,4-tetrahydro-acridin-2-ol, Hydrochlorid;
*rac*-cis-[4-Dimethylaminomethyl-3-(3-methoxy-phenyl)]-1,2,3,4-tetrahydro-acridin-3-ol, Hydrochlorid;
[3-Dimethylaminomethyl-2-(3-hydroxy-phenyl)]-3,4-dihydro-acridin-1-en, Hydrochlorid;
*rac*-trans-[3-Dimethylaminomethyl-2-(3-methoxy-phenyl)]-1,2,3,4-tetrahydro-acridin-2-ol, Hydrochlorid;
*rac-*cis-[3-Dimethylaminomethyl-2-(3-hydroxy-phenyl)]-1,2,3,4-tetrahydro-acridin-2-ol, Hydrochlorid;
[1-(3-Methoxy-phenyl)-3,4-dihydro-acridin-2-ylmethyl]-dimethylamin, Hydrochlorid;
[3-(3-Methoxy-phenyl)-1,2-dihydro-acridin-2-ylmethyl]-dimethylamin, Hydrochlorid;
[3-Dimethylaminomethyl-2-(3-methoxy-phenyl)]-3,4-dihydro-acridin-1-en, Hydrochlorid;
*rac*-trans-[1-(3-Methoxy-phenyl)-1,2,3,4-tetrahydroacridin-2-yl-methyl]-dimethylamin, Hydrochlorid;
*rac*-cis-[1-(3-Methoxy-phenyl)-1,2,3,4-tetrahydroacridin-2-yl-methyl]-dimethylamin, Hydrochlorid;
*rac*-trans-[3-(3-Methoxy-phenyl)-1,2,3,4-tetrahydroacridin-2-yl-methyl]-dimethylamin, Hydrochlorid;
*rac*-cis-[3-(3-Methoxy-phenyl)-1,2,3,4-tetrahydroacridin-2-yl-methyl]-dimethylamin, Hydrochlorid;
[3-(2-Dimethylaminomethyl-3,4-dihydro-acridin-1-yl)]-phenol, Hydrochlorid;
[3-(2-Dimethylaminomethyl-1,2-dihydro-acridin-3-yl]-phenol;
*rac*-trans-[3-(2-Dimethylaminomethyl-1,2,3,4-tetrahydro-acridin-3-yl)]-phenol;
*rac*-trans-[3-(2-Dimethylaminomethyl-1,2,3,4-tetrahydro-acridin-1-yl)]-phenol, Hydrochlorid;
*rac*-cis-[2-Dimethylaminomethyl-1-(3-methoxy-phenyl)]-3,3-dimethyl-1,2,3,4-tetrahydro-acridin-1-ol, Hydrochlorid;
3-(2-Dimethylaminomethyl-3,3-dimethyl-3,4-dihydroacridin-1-yl)-phenol, Hydrochlorid.

8. Verfahren zur Herstellung von Acridinderivaten der allgemeinen Formel (I) 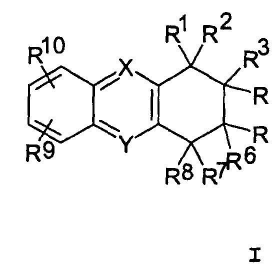 worin
R¹ bis R¹⁰ die Bedeutung gemäß Anspruch 1 haben, wobei Derivate ausgeschlossen sind, in denen die Reste die folgende Bedeutung haben, daß R¹ A, R² H, OR¹² oder R² und R³ zusammen eine Doppelbindung bilden, R³ H oder R³ und R² zusammen eine Doppelbindung bilden, R⁴ CH₂NR¹⁴R¹⁵, R⁵ und R⁶ C₁₋₆-Alkyl, R⁷ und R⁸ H, und die Reste R¹¹, R¹², R¹⁴ und R¹⁵ die gleiche Bedeutung wie in Anspruch 1 haben, **dadurch gekennzeichnet, daß** Cyclohexanderivate der allgemeinen Formeln II, III, oder IV 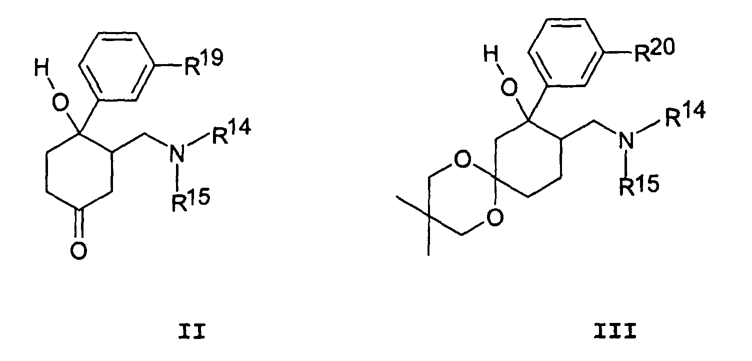 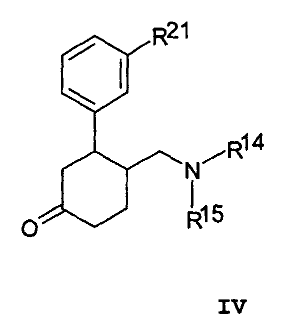 worin
R¹⁹, R²⁰ und R²¹ unabhängig voneinander H, C₁₋₆₋Alkoxy, O-C₃₋₇-Cycloalkyl; 0-Aryl, wobei "Aryl" unsubstituiertes oder ein- oder mehrfach mit OH, F, Cl, CF₃, C₁₋₆-Alkyl, C₁₋₆-Alkoxy, C₃₋₇-Cycloalkyl, C₂₋₆-Alkylen, Heterocyclyl oder Phenyl substituiertes Phenyl oder Naphthyl bedeutet; oder O-Heterocyclyl, wobei "Heterocyclyl" 5- oder 6-gliedrige gesättigte oder ungesättigte, gegebenenfalls mit einem ankondensierten Arylsystem versehene, heterocyclische Verbindungen bedeutet, die ein oder zwei Heteroatome aus der Gruppe von Stickstoff, Sauerstoff und/oder Schwefel enthalten; sowie R¹⁴ und R¹⁵ die gleiche Bedeutung wie in Anspruch 1 besitzen, mit substituierten 2-Aminobenzaldehyden in einem Lösemittel aus der Gruppe von Essigsäureethylester oder von C₁₋₄-Alkylalkohol in Gegenwart einer Säure umgesetzt werden und die Eliminierung der tertiären OH-Gruppe und/oder Spaltung der Methylether-Gruppierung in den erhaltenen Cyclisierungsprodukten durch Umsetzung der Produkte mit einer Säure zu Acridinderivaten der allgemeinen Formel I durchgeführt wird.

9. Verwendung der Acridinderivate der allgemeinen Formel I nach Anspruch 1 als Wirkstoff in einem Arzneimittel.

10. Verwendung nach Anspruch 9, **dadurch gekennzeichnet, daß** das Arzneimittel ein Analgetikum ist.

## Claims

1. Acridine derivative corresponding to formula I 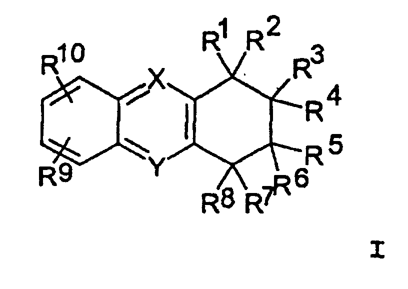 wherein
R¹ denotes A, if
R² denotes H or OR¹², or R² and R³ together form a double bond,
R³ denotes H, or R³ and R² together form a double bond,
R⁴ denotes CH₂NR¹⁴R¹⁵;
R⁵ denotes H or C₁₋₆ alkyl;
R⁶ denotes H or C₁₋₆ alkyl;
R⁷ denotes H;
R⁸ denotes H;
or
R³ denotes A, if
R¹ denotes H or R¹ and R⁴ together form a double bond;
R² denotes H;
R⁴ denotes H, OR¹², or R⁴ and R¹ together form a double bond or R⁴ and R⁵ together form a double bond;
R⁵ denotes H, or R⁵ and R⁴ together form a double bond;
R⁶ denotes CH₂NR¹⁴ R¹⁵;
R⁷ denotes H;
R⁸ denotes H;
or
R⁵ denotes A, if
R¹ denotes H;
R² denotes H;
R³ denotes H, or R³ and R⁶ together form a double bond;
R⁴ denotes H;
R⁶ denotes H, OR¹², or R⁶ and R³ together form a double bond, or R⁶ and R⁷ together form a double bond;
R⁷ denotes H, or R⁷ and R⁶ together form a double bond;
R⁸ denotes CH₂NR¹⁴R¹⁵;
and
A denotes 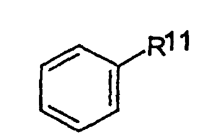
R⁹, R¹⁰ are identical or different from each other and denote H, OH, C₁₋₆ alkoxy, Cl, F, CF₃, CN, COOH, CONR¹⁷R¹⁸ or COOR¹⁶;
R¹¹ denotes H, OH, C₁₋₆ alkoxy, O-C₃₋₇ cycloalkyl, O-aryl, wherein "aryl" denotes unsubstituted phenyl or naphthyl or phenyl or naphthyl singly or multiply substituted by OH, F, Cl, CF₃, C₁₋₆-alkyl, C₁₋₆-alkoxy, C₃₋₇-cycloalkyl, C₂₋₆-alkylene, heterocyclyl or phenyl; O-heterocyclyl, wherein "heterocyclyl" denotes 5- or 6-membered saturated or unsaturated heterocyclic compounds, which optionally comprise an aryl system which is incorporated by condensation, and which contain one or two heteroatoms from the group consisting of nitrogen, oxygen and/or sulphur;
R¹² denotes H, C₁₋₆ alkyl; aryl, wherein "aryl" denotes unsubstituted phenyl or naphthyl or phenyl or naphthyl singly or multiply substituted by OH, F, Cl, CF₃, C₁₋₆-alkyl, C₁₋₆ alkoxy, C₃₋₇-cycloalkyl, C₂₋₆-alkylene, heterocyclyl or phenyl; COR¹³
R¹³ denotes C₁₋₆ alkyl; aryl, wherein "aryl" denotes unsubstituted phenyl or naphthyl or phenyl or naphthyl singly or multiply substituted by OH, F, Cl, CF₃, C₁₋₆-alkyl, C₁₋₆ alkoxy, C₃₋₇-cycloalkyl, C₂₋₆-alkylene, heterocyclyl or phenyl;
R¹⁴, R¹⁵ are identical or different from each other and denote C₁₋₆ alkyl; aryl, wherein "aryl" denotes unsubstituted phenyl or naphthyl or phenyl or naphthyl singly or multiply substituted by OH, F, Cl, CF₃, C₁₋₆-alkyl, C₁₋₆ alkoxy, C₃₋₇-cycloalkyl, C₂₋₆-alkylene, heterocyclyl or phenyl; C₃₋₇ cycloalkyl;
R¹⁶ denotes C₁₋₆ alkyl; aryl, wherein "aryl" denotes unsubstituted phenyl or naphthyl or phenyl or naphthyl singly or multiply substituted by OH, F, Cl, CF₃, C₁₋₆-alkyl, C₁₋₆ alkoxy, C₃₋₇-cycloalkyl, C₂₋₆-alkylene, heterocyclyl or phenyl;
R¹⁷, R¹⁸ are identical or different from each other and denote C₁₋₆ alkyl; aryl, wherein "aryl" denotes unsubstituted phenyl or naphthyl or phenyl or naphthyl singly or multiply substituted by OH, F, Cl, CF₃, C₁₋₆-alkyl, C₁₋₆ alkoxy, C₃₋₇-cycloalkyl, C₂₋₆-alkylene, heterocyclyl or phenyl; and
X represents N if Y represents C, or X represents C if Y represents N,
or a pharmaceutically acceptable salts thereof.

2. Compound according to claim 1, **characterised in that** R¹⁴ and R¹⁵ are identical or different and denote C₁₋₆ alkyl.

3. Compound according to claim 1, **characterised in that** R¹¹ denotes OH or C₁₋₆ alkoxy.

4. Compound according to claim 1, **characterised in that** R¹ denotes A, R¹¹ denotes OH or C₁₋₆ alkoxy, and R¹⁴ and R¹⁵, independently of each other, denote C₁₋₆ alkyl.

5. Compound according to claim 1, **characterised in that** R³ denotes A, R¹¹ denotes OH or C₁₋₆ alkoxy, and R¹⁴ and R¹⁵, independently of each other, denote C₁₋₆ alkyl.

6. Compound according to claim 1, **characterised in that** R⁵ denotes A, R¹¹ denotes OH or C₁₋₆ alkoxy, and R¹⁴ and R¹⁵, independently of each other, denote C₁₋₆ alkyl.

7. Compounds according to claim 1, selected from the group consisting of:
*rac*-cis-[3-dimethylaminomethyl-2-(3-methoxy-phenyl)]-1,2,3,4-tetrahydro-acridin-2-ol hydrochloride;
*rac*-cis-[4-dimethylaminomethyl-3-(3-methoxy-phenyl)]-1,2,3,4-tetrahydro-acridin-3-ol hydrochloride;
[3-dimethylaminomethyl-2-(3-hydroxy-phenyl)]-3,4-dihydro-acridin-1-ene hydrochloride;
*rac*-trans-[3-dimethylaminomethyl-2-(3-methoxyphenyl)]-1,2,3,4-tetrahydro-acridin-2-ol hydrochloride;
*rac*-cis-[3-dimethylaminomethyl-2-(3-hydroxyphenyl)]-1,2,3,4-tetrahydro-acridin-2-ol hydrochloride;
[1-(3-methoxy-phenyl)-3,4-dihydro-acridin-2-yl-methyl]-dimethylamine hydrochloride;
[3-(3-methoxy-phenyl)-1,2-dihydro-acridin-2-yl-methyl]-dimethylamine hydrochloride;
[3-dimethylaminomethyl-2-(3 -methoxy-phenyl)]-3,4-dihydro-acridin-1-ene hydrochloride;
*rac*-trans-[1-(3-methoxy-phenyl)-1,2,3,4-tetrahydro-acridin-2-yl-methyl]-dimethylamine hydrochloride;
*rac*-cis-[1-(3 -methoxy -phenyl)-1,2,3,4-tetrahydro -acridin -2 -yl -methyl]-dimethylamine hydrochloride;
*rac*-trans-[3-(3-methoxy-phenyl)-1,2,3,4-tetrahydro-acridin-2-yl-methyl]-dimethylamine hydrochloride;
*rac*-cis-[3-(3-methoxy-phenyl)-1,2,3,4-tetrahydro-acridin-2-yl-methyl]-dimethylamine hydrochloride;
[3-(2-dimethylaminomethyl-3,4-dihydro-acridin-1-yl)]-phenol hydrochloride;
[3-(2-dimethylaminomethyl-1,2-dihydro-acridin-3-yl]-phenol;
*rac*-trans-[3-(2-dimethylaminomethyl-1,2,3,4-tetra-hydro-acridin-3-yl)]-phenol;
*rac*-trans-[3-(2-dimethylaminomethyl-1,2,3,4-tetra-hydro-acridin-1-yl)]-phenol hydrochloride;
*rac*-cis-[2-dimethylaminomethyl-1-(3-methoxy-phenyl]-3,3-dimethyl-1,2,3,4-tetrahydro-acridin-1-ol hydrochloride; and
3-(2-dimethylaminomethyl-3,3-dimethyl-3,4-dihydro-acridin-1-yl)-phenol hydrochloride.

8. Method of producing acridine derivatives corresponding to formula (I) 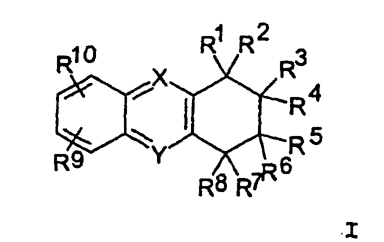 wherein R¹ to R¹⁰ have the meanings according to claim 1, wherein derivatives are excluded in which the radicals have the following meanings: R¹ denotes A, R² denotes H or OR¹²,or R² and R³ together form a double bond, R³ denotes H, or R³ and R² together form a double bond, R⁴ denotes CH₂NR¹⁴R¹⁵, R⁵ and R⁶ denote C₁₋₆ alkyl, R⁷ and R⁸ denote H, and R¹¹, R¹², R¹⁴ and R¹⁵ have the same meanings as in claim 1, **characterised in that** cyclohexane derivatives corresponding to general formulae II, III, or IV 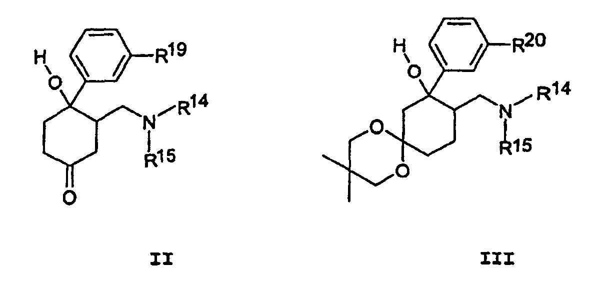 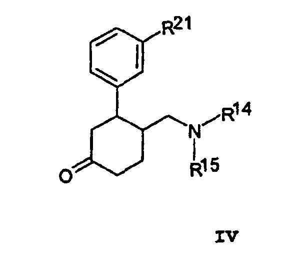 wherein
R¹⁹, R²⁰ and R²¹, independently of each other, represent H, C₁₋₆ alkoxy, O-C₃₋₇-ycloalkyl; O-aryl, wherein "aryl" denotes unsubstituted phenyl or naphthyl or phenyl or naphthyl singly or multiply substituted by OH, F, Cl, CF₃, C₁₋₆ alkyl, C₁₋₆ alkoxy, C₃₋₇ cycloalkyl, C₂₋₆ alkylene, heterocyclyl or phenyl or O-heterocyclyl, wherein "heterocyclyl" denotes 5- or 6-membered, saturated or unsaturated heterocyclic compounds which optionally comprise an aryl system which is incorporated by condensation, and contain one or two heteroatoms from the group consisting of nitrogen, oxygen and/or sulphur; and R¹⁴ and R¹⁵ have the same meanings as in claim 1, are reacted with substituted 2-aminobenzaldehydes in a solvent selected from the group consisting of ethyl acetate and C₁₋₄ alkyl alcohols in the presence of an acid, and eliminating the tertiary OH group and/or cleaving the methyl ether group in the cyclisation products obtained by reacting the products with an acid to form acridine derivatives of general formula I.

9. Use of the acridine derivatives of general formula I according to claim 1 as an active ingredient in a pharmaceutical composition.

10. Use according to claim 9, **characterised in that** the pharmaceutical composition is an analgesic.

## Revendications

1. Dérivés d'acridine de formule générale I, 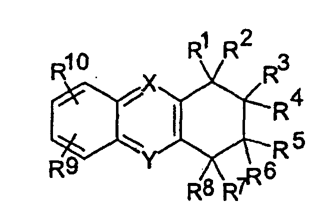 dans laquelle
R¹ signifie A quand
R² signifie H, OR¹², ou R² et R³ forment ensemble une double liaison ;
R³ signifie H, ou R³ et R² forment ensemble une double liaison ;
R⁴ signifie CH₂NR¹⁴R¹⁵ ;
R⁵ signifie H, un groupe alkyle en C₁₋₆ ;
R⁶ signifie H, un groupe alkyle en C₁₋₆ ;
R⁷ signifie H ;
R⁸ signifie H ;
ou
R³ signifie A quand
R¹ signifie H, ou R¹ et R⁴ forment ensemble une double liaison ;
R² signifie H ;
R⁴ signifie H, OR¹², ou R⁴ et R¹ forment ensemble une double liaison, ou R⁴ et R⁵ forment ensemble une double liaison ;
R⁵ signifie H, ou R⁵ et R⁴ forment ensemble une double liaison ;
R⁶ signifie CH₂NR¹⁴R¹⁵ ;
R⁷ signifie H ;
R⁸ signifie H ;
ou
R⁵ signifie A quand
R¹ signifie H ;
R² signifie H ;
R³ signifie H, ou R³ et R⁶ forment ensemble une double liaison ;
R⁴ signifie H ;
R⁶ signifie H, OR¹², ou R⁶ et R³ forment ensemble une double liaison, ou R⁶ et R⁷ forment ensemble une double liaison ;
R⁷ signifie H, ou R⁷ et R⁶ forment ensemble une double liaison ;
R⁸ signifie CH₂NR¹⁴R¹⁵ ;
et
A signifie 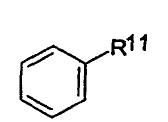
R⁹, R¹⁰ sont identiques ou différents l'un de l'autre et signifient H, OH, un groupe alkoxy en C₁₋₆, Cl, F, CF₃, CN, COOH, CONR¹⁷R¹⁸, COOR¹⁶ ;
R¹¹ signifie H, OH, un groupe alkoxy en C₁₋₆, O-cycloalkyle en C₃₋₇ ; O-aryle, le groupe "aryle" signifiant un groupe phényle ou naphtyle non substitué ou mono- ou polysubstitué par un groupe OH, F, Cl, CF₃, alkyle en C₁₋₆, alkoxy en C₁₋₆, cycloalkyle en C₃₋₇, alkylène en C₂₋₆, hétérocyclyle ou phényle ; O-hétérocyclyle, le groupe "hétérocyclyle" signifiant des composés hétérocycliques à 5 ou 6 chaînons, saturés ou insaturés, éventuellement pourvus d'un système aryle condensé, qui contiennent un ou deux hétéroatomes choisis dans l'ensemble constitué de l'azote, de l'oxygène et/ou du soufre ;
R¹² signifie H, un groupe alkyle en C₁₋₆ ; aryle, le groupe "aryle" signifiant un groupe phényle ou naphtyle non substitué ou mono- ou polysubstitué par un groupe OH, F, Cl, CF₃, alkyle en C₁₋₆, alkoxy en C₁₋₆, cycloalkyle en C₃₋₇, alkylène en C₂₋₆, hétérocyclyle ou phényle ; COR¹³ ;
R¹³ signifie un groupe alkyle en C₁₋₆ ; aryle, le groupe "aryle" signifiant un groupe phényle ou naphtyle non substitué ou mono- ou polysubstitué par un groupe OH, F, Cl, CF₃, alkyle en C₁₋₆, alkoxy en C₁₋₆, cycloalkyle en C₃₋₇, alkylène en C₂₋₆, hétérocyclyle ou phényle ;
R¹⁴, R¹⁵ sont identiques ou différents l'un de l'autre et signifient un groupe alkyle en C₁₋₆ ; aryle, le groupe "aryle" signifiant un groupe phényle ou naphtyle non substitué ou mono- ou polysubstitué par un groupe OH, F, Cl, CF3, alkyle en C₁₋₆, alkoxy en C₁₋₆, cycloalkyle en C₃₋₇, alkylène en C₂₋₆, hétérocyclyle ou phényle ; cycloalkyle en C₃₋₇ ;
R¹⁶ signifie un groupe alkyle en C₁₋₆ ; aryle, le groupe "aryle" signifiant un groupe phényle ou naphtyle non substitué ou mono- ou polysubstitué par un groupe OH, F, Cl, CF₃, alkyle en C₁₋₆, alkoxy en C₁₋₆, cycloalkyle en C₃₋₇, alkylène en C₂₋₆, hétérocyclyle ou phényle ;
R¹⁷, R¹⁸ sont identiques ou différents l'un de l'autre et signifient un groupe alkyle en C₁₋₆ ; aryle, le groupe "aryle" signifiant un groupe phényle ou naphtyle non substitué ou mono- ou polysubstitué par un groupe OH, F, Cl, CF₃, alkyle en C₁₋₆, alkoxy en C₁₋₆, cycloalkyle en C₃₋₇, alkylène en C₂₋₆, hétérocyclyle ou phényle ; et
X = N quand Y = C, ou X = C quand Y = N,
ou des sels pharmaceutiquement acceptables de ceux-ci.

2. Composés selon la revendication 1, **caractérisés en ce que** R¹⁴ et R¹⁵, identiques ou différents l'un de l'autre, signifient un groupe alkyle en C₁₋₆.

3. Composés selon la revendication 1, **caractérisés en ce que** R¹¹ signifie OH ou un groupe alkoxy en C₁₋₆.

4. Composés selon la revendication 1, **caractérisés en ce que** R¹ signifie A, R¹¹ signifie OH ou un groupe alkoxy en C₁₋₆, R¹⁴ et R¹⁵ signifient, indépendamment l'un de l'autre, un groupe alkyle en C₁₋₆.

5. Composés selon la revendication 1, **caractérisés en ce que** R³ signifie A, R¹¹ signifie OH ou un groupe alkoxy en C₁₋₆, R¹⁴ et R¹⁵ signifient, indépendamment l'un de l'autre, un groupe alkyle en C₁₋₆.

6. Composés selon la revendication 1, **caractérisés en ce que** R⁵ signifie A, R¹¹ signifie OH ou un groupe alkoxy en C₁₋₆, R¹⁴ et R¹⁵ signifient, indépendamment l'un de l'autre, un groupe alkyle en C₁₋₆.

7. Composés selon la revendication 1, choisis dans l'ensemble constitué de :
*rac*-cis-[3-diméthylaminométhyl-2-(3-méthoxyphényl)]-1,2,3,4-tétrahydro-acridine-2-ol, chlorhydrate ;
*rac*-cis-[4-diméthylaminométhyl-3-(3-méthoxyphényl)]-1,2,3,4-tétrahydro-acridine-3-ol, chlorhydrate ;
[3-diméthylaminométhyl-2-(3-hydroxyphényl)]-3,4-dihydro-acridine-1-ène, chlorhydrate ;
*rac*-trans-[3-diméthylaminométhyl-2-(3-méthoxyphényl)]-1,2,3,4-tétrahydro-acridine-2-ol, chlorhydrate ;
*rac*-cis-[3-diméthylaminométhyl-2-(3-hydroxyphényl)]-1,2,3,4-tétrahydro-acridine-2-ol, chlorhydrate ;
[1-(3-méthoxyphényl-)-3,4-dihydro-acridine-2-yl-méthyl]-diméthylamine, chlorhydrate ;
[3-(3-méthoxyphényl)-1,2-dihydro-acridine-2-yl-méthyl]-diméthylamine, chlorhydrate ;
[3-diméthylaminométhyl-2-(3-méthoxyphényl)]-3,4-dihydro-acridine-1-ène, chlorhydrate ;
*rac*-trans-[1-(3-méthoxyphényl)-1,2,3,4-tétrahydro-acridine-2-yl-méthyl]-diméthylamine, chlorhydrate ;
*rac*-cis-[1-(3-méthoxyphényl)-1,2,3,4-tétrahydro-acridine-2-yl-méthyl]-diméthylamine, chlorhydrate ;
*rac*-trans-[3-(3-méthoxyphényl)-1,2,3,4-tétrahydro-acridine-2-yl-méthyl]-diméthylamine, chlorhydrate ;
*rac*-cis-[3-(3-méthoxyphényl)-1,2,3,4-tétrahydro-acridine-2-yl-méthyl]-diméthylamine, chlorhydrate ;
[3-(2-diméthylaminométhyl-3,4-dihydro-acridine-1-yl)]-phénol, chlorhydrate ;
[3-(2-diméthylaminométhyl-1,2-dihydro-acridine-3-yl)]-phénol ;
*rac*-trans-[3-(2-diméthylaminométhyl-1,2,3,4-tétrahydroacridine-3-yl)]-phénol ;
*rac*-trans-[3-(2-diméthylaminométhyl-1,2,3,4-tétrahydroacridine-1-yl)]-phénol, chlorhydrate ;
*rac*-cis-[2-diméthylaminométhyl-1-(3-méthoxyphényl)]-3,3-diméthyl-1,2,3,4-tétrahydro-acridine-1-ol, chlorhydrate ;
[3-(2-diméthylaminométhyl-3,3-diméthyl-3,4-dihydroacridine-1-yl)]-phénol, chlorhydrate.

8. Procédé de préparation de dérivés d'acridine de formule générale (I) 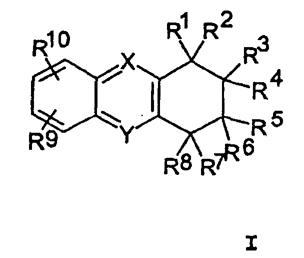 dans laquelle
R¹ à R¹⁰ ont la signification selon la revendication 1, à l'exclusion des dérivés dans lesquels les radicaux ont la signification suivante : R¹ signifie A, R² signifie H, OR¹² ou R² et R³ forment ensemble une double liaison, R³ signifie H et R³ et R² forment ensemble une double liaison, R⁴ signifie CH₂NR¹⁴R¹⁵, R⁵ et R⁶ signifient un groupe alkyle en C₁₋₆, R⁷ et R⁸ signifient H et les radicaux R¹¹, R¹², R¹⁴ et R¹⁵ ont la même signification que dans la revendication 1, **caractérisé en ce qu'**on fait réagir des dérivés de cyclohexane de formules générales II, III ou IV, 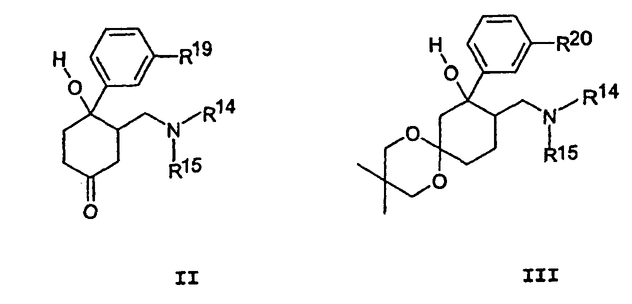 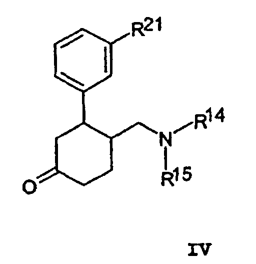 dans lesquelles
R¹⁹, R²⁰ et R²¹ signifient, indépendamment les uns des autres, H, un groupe alkoxy en C₁₋₆, O-cycloalkyle en C₃₋₇ ; O-aryle, "aryle" signifiant un groupe phényle ou naphtyle non substitué ou mono- ou polysubstitué par un groupe OH, F, Cl, CF₃, alkyle en C₁₋₆, alkoxy en C₁₋₆, cycloalkyle en C₃₋₇, alkylène en C₂₋₆, hétérocyclyle ou phényle ; ou O-hétérocyclyle, "hétérocyclyle" signifiant des composés hétérocycliques à 5 ou 6 chaînons, saturés ou insaturés, éventuellement pourvus d'un système aryle condensé, qui contiennent un ou deux hétéroatomes choisis dans l'ensemble constitué de l'azote, de l'oxygène et/ou du soufre ; et R¹⁴ et R¹⁵ ont la même signification que dans la revendication 1, avec des 2-aminobenzaldéhydes substitués, dans un solvant choisi dans l'ensemble constitué de l'acétate d'éthyle ou d'un alcool d'alkyle en C₁₋₄, en présence d'un acide, et **en ce qu'**on effectue l'élimination du groupe OH tertiaire et/ou la séparation du groupement éther méthylique dans les produits de cyclisation obtenus par réaction des produits avec un acide pour obtenir des dérivés d'acridine de formule générale I.

9. Utilisation des dérivés d'acridine de formule générale I selon la revendication 1 en tant que principe actif dans un médicament.

10. Utilisation selon la revendication 9, **caractérisée en ce que** le médicament est un analgésique.
